# FASCICULE DE BREVET EUROPEEN

(11) **EP 0 646 009 B1**
(45) Date de publication et mention de la délivrance du brevet: **12.08.1998**
(21) Numéro de dépôt: 93913178.5
(22) Date de dépôt: 23.06.1993
(51) Int. Cl.: A61K 31/70, A61K 31/52

(54) **UTILISATION DE LA GUANOSINE POUR LA FABRICATION DE MEDICAMENTS DESTINES A TRAITER LES DEFICITS FONCTIONNELS CEREBRAUX**
VERWENDUNG VON GUANOSIN ZUR HERSTELLUNG VON ARZNEIMITTELN ZUR BEHANDLUNG VON ZEREBRALEN FUNKTIONSSTÖRUNGEN
USE OF GUANOSINE IN THE MANUFACTURE OF DRUGS FOR THE TREATMENT OF BRAIN DYSFUNCTION

(30) Priorité: 24.06.1992 FR 9207721
(43) Date de publication de la demande: 05.04.1995
(73) Titulaire: PIERRE FABRE MEDICAMENT, 92100 Boulogne (FR)
(72) Inventeur: PEETERS, Marie A., F-75015 Paris (FR); LEJEUNE, Jerôme, F-75005 Paris (FR)
(74) Mandataire: Ahner, Francis
(86) Numéro de dépôt international: FR9300625
(87) Numéro de publication internationale: WO9400132

(56) Documents cités:
- EP-A- 0 355 899
- EP-A- 0 470 317
- FR-A- 2 437 834
- BRAIN DYSFUNCTION vol. 5, no. 5-6, 1992, pages 288 - 300 PEETERS, M.A. ET AL 'FRAGILE X SYNDROME: A POSSIBLE DEFECT OF GUANOSINE PATHWAY'
- MONATSSCHRIFT KINDERHEILKUNDE vol. 139, no. 10, Octobre 1991, pages 655 - 661 LEJEUNE, JEROME 'ZUR PATHOGENESE DER DEBILIT[T BEI DER TRISOMIE 21'
- TRENDS IN PHARMACOLOGICAL SCIENCE vol. 11, no. 9, Septembre 1991, pages 342 - 343 IJZERMAN, A.D. ET AL 'PHARMACOLOGY OF PURINERGIC RECEPTORS: IMPLICATIONS FOR DRUG DESIGN'

## Description

La présente invention concerne le domaine des médicaments destinés au traitement de divers déficits fonctionnels cérébraux.

Dans le cadre d'une analyse générale de la pathogénie des maladies métaboliques déterminant une débilité de l'intelligence, un certain nombre de mécanismes chimiques indispensables au fonctionnement des neurones ont été analysés. Parmi ceux-ci, il semble que le métabolisme des monocarbonnés joue un rôle primordial. En effet, la plupart des blocages enzymatiques provoquant une débilité de l'intelligence, sans lésion primaire des constituants des membranes et des gaines et sans trouble majeur de l'embryogenèse cérébrale, présentent en commun un trouble des monocarbonnés.

C'est ainsi que des dosages in vitro examinant la variation de l'indice mitotique en présence de divers métabolites et antimétabolites de la synthèse des purines, ont permis d'établir une corrélation très significative avec divers syndromes chromosomiques. En particulier, il a ainsi pu être mis en évidence une augmentation très significative de l'indice mitotique de lymphocytes provenant de malades atteints du syndrome de l'X fragile, lorsqu'on ajoute au milieu de culture de la guanosine ou encore un précurseur ou un dérivé de la guanosine.

C'est pourquoi la présente invention concerne l'utilisation de la guanosine pour la fabrication de médicaments destinés au traitement des déficits fonctionnels cérébraux.

Les médicaments entrant dans le cadre de la présente invention sont en particulier destinés à assurer le traitement des syndromes chromosomiques entraînant une débilité de l'intelligence, telle que la trisomie 21 et la fragilité de l'X, ainsi que des troubles du comportement survenant au cours de maladies du système nerveux central, telle que la maladie d'Alzheimer. Ces médicaments peuvent également être destinés au traitement de syndromes d'hyperactivité, d'hyperréactivité, d'anxiété, ou de réactions psychotiques ou autistiques.

D'autres caractéristiques et avantages de la présente invention apparaîtront à la lecture de la description détaillée faite ci-après, notamment en regard de quelques résultats expérimentaux.

Le syndrome de l'X fragile est la seconde cause chromosomique du retard mental et la cause la plus commune du retard mental familial. Le taux de mutation est élevé. L'incidence calculée est de 1 sur 1250 chez les hommes et de 1 sur 2000 chez les femmes. Ainsi, un diagnostic précis et précoce est important en vue d'un traitement spécifique.

Ce syndrome se caractérise par la présence d'un site fragile sur le bras long du chromosome X, en Xq27. Le retard mental est transmis selon le mode dominant lié à l'X avec pénétrance incomplète. Environ 30% des porteurs féminins hétérozygotes ont un retard mental et environ 20% des hommes transmetteurs ne présentent ni le site fragile, ni les symptômes cliniques. Le génotype et le phénotype ne sont donc pas en corrélation absolue.

Il a été observé que l'expression de ce site fragile in vitro dépendait de la composition du milieu de culture et que les déficiences en certains composés puriques pourraient favoriser l'expression du site fragile dans les lymphocytes.

Il a ainsi pu être déterminé qu'un défaut du métabolisme purique, qu'il soit primaire ou secondaire, pouvait jouer un rôle majeur dans divers syndromes du retard mental.

### Exemple d'expérimentation in vitro avec la guanosine

On cultive vingt-huit échantillons de sang périphérique provenant de malades atteints du syndrome de l'X fragile (vingt-quatre hommes et quatre femmes retardés) pendant 72 heures dans le milieu TC199 (Seromed^{R}) additionné de sérum AB humain à 25%, phytohémagglutine C (IBF, France), pénicilline et streptomycine. La technique de culture, la récolte et l'examen microscopique sont effectués de manière classique en soi connue.

On ajoute également au début de la culture de la guanosine à une concentration de 3,1 mg/l.

On recueille les cultures lymphocytaires avec les techniques standards utilisées pour l'analyse chromosomique. On colore les lames au Giemsa et on les code. On fait lire un minimum de 3000 cellules par deux observateurs différents pour calculer l'indice mitotique, exprimé sous la forme du rapport : nombre de mitoses / nombre de cellules.

Vingt-six malades ne reçoivent pas de médicament au moment de la culture des lymphocytes.

Les témoins utilisés pour l'analyse sont les suivants : 135 adultes normaux, 79 malades atteints de trisomie 21, 10 malades atteints du syndrome du cri du chat et 68 malades atteints de retard mental (avec ou sans anomalies chromosomiques autres que celles mentionnées ci-dessus).

On analyse les résultats en comparant l'indice mitotique de chaque expérience à la propre culture témoin du malade et on exprime les résultats en pourcentage d'augmentation ou de diminution en pourcentage de l'indice mitotique. Les comparaisons statistiques entre les groupes se font sur la base du test t de Student. Les résultats de l'expérimentation in vitro sont consignés dans les tableaux I à III ci-après.

Il apparait ainsi que l'addition de guanosine augmente de façon significative l'indice mitotique chez les malades atteints du syndrome de l'X fragile. Lorsqu'on compare à l'échantillon total de malades (malades atteints de retard mental de diverses étiologies et malades atteints du syndrome de Down), la différence est très significative (0,01<p<0,005); lorsqu'on compare au total des témoins, la différence n'est pas aussi marquée mais reste significative (0,05<p<0,025). Ceci semble être dû aux différences entre la réponse des hommes et des femmes à la guanosine (les témoins femmes augmentant leur indice mitotique significativement plus que les malades témoins masculins : p = 0,025). On affine donc l'analyse pour examiner les réponses chez les malades masculins seulement. On observe ainsi une différence très significative dans la réponse à la guanosine lorsqu'on compare les hommes atteints de l'X fragile à l'échantillon total des hommes (p=0,001), aux témoins masculins normaux (0,005<p<0,001) ou aux hommes ayant des retards d'autres étiologies (0,005<p<0,001). La différence entre les hommes atteints de l'X fragile et les hommes atteints du syndrome de Down est significative (p = 0,025).

Pour tester la spécificité des modifications observées, on recherche les malades manifestant une augmentation de l'indice mitotique supérieure à 10% en présence de guanosine.
- 13/19 malades atteints d'X fragile présentent une augmentation de plus de 10% en présence de guanosine (tous les malades atteints de l'X fragile augmentent leur indice mitotique).
- 29/80 témoins normaux (X = 6,5; 0,02<p<0,01)
- 7/29 témoins masculins normaux (X=9,3; 0,01<<p<0,001)
- 12/34 malades atteints de trisomie 21 (X=5,4; p=0,02). Il y a 7 femmes et 5 hommes; les 5 hommes ont tous des complications psychotiques.
- 17/58 malades atteints de retard mental (X=9,2; 0,01<<p<0,0001).

Il y a 7 femmes et 10 hommes. Il faut noter que 9 des 10 malades masculins présentent des complications psychiatriques associées. Plusieurs hommes présentant une augmentation de l'indice mitotique en présence de guanosine avaient été adressés à l'origine en vue d'une recherche de Xqfra, sans que l'examen cytogénétique ait décelé une fragilité de l'X.

Des tests de fonctionnement thyroïdien chez des hommes atteints du syndrome de l'X fragile et chez des hommes atteints de retard mental lié à l'X ont montré des niveaux sériques basaux normaux d'hormones thyroïdiennes et de TSH. La réponse à la TRH est moins nette que chez les sujets normaux. Etant donné que la liaison au récepteur THR peut être réglée de façon allostérique par les nucléotides de guanine, on peut raisonnablement faire l'hypothèse que, si les hommes atteints d'X fragile présentent un certain défaut sur la voie de la guanine, ceci pourrait modifier la liaison en récepteur de THR et par conséquent la réponse à TSH.

L'effet des nucléotides de guanine sur les neurotransmetteurs du système nerveux central est extrêmement important. On ne sait pas si la guanosine est un neurotransmetteur comme l'adénosine, mais on sait que les nucléotides de guanine modulent négativement la liaison agoniste aux récepteurs adrénergiques à la dopamine, muscarinique-cholinergique et aux opiacées. Les récepteurs à la phéncyclidine (PCP) sont également régulés par les nucléotides de guanine. En outre, les nucléotides de guanine jouent un rôle majeur dans le métabolisme de la ptérine (GPT et le précurseur de la tétrahydrobioptérine), ainsi que dans la dynamique des microtubules (synthèse de la tubuline).

Le cAMP et le cGMP jouent un rôle unique dans la régulation des neurotransmetteurs et leur rapport semble être d'une importance clinique. De fait, il a été suggéré que ces deux nucléotides cycliques régulent l'équilibre cholénergique-adrénergique.

Après la naissance, le métabolisme purique dans le système nerveux central est caractérisé par une diminution de la synthèse purique de novo, par une augmentation de l'activité d'hypoxanthinephosphoribosyltransférase (HPRT) et par une absence d'activité de la xanthine oxydase. Par conséquent, le cerveau est largement dépendant des voies de récupération pour conserver le niveau de GTP. Le métabolisme essentiellement de novo pendant le développement du cerveau (croissance des neuroblastes et prolifération neurogliale) pourrait expliquer pourquoi les malades atteints d'X fragile ont une morphologie cérébrale sensiblement normale. La principale modification neuroanatomique documentée chez ces malades (ainsi que chez les malades atteints d'autisme) est l'hypoplasie du vermis postérieur.

Tout défaut métabolique le long de la voie de récupération des purines aurait des conséquences neurologiques et comportementales manifestes après cette période de prolifération neuroblastique et neurogliale. La période périnatale pendant laquelle la permutation génique et l'impression des récepteurs se produit est sensible à une "tératogénèse métabolique" tardive. Un défaut métabolique génétique faisant intervenir un gène ou un groupe de gènes normalement activés pendant cette période aura des répercussions importantes sur les récepteurs membranaires et sur le développement post-natal du cerveau. Il est intéressant de noter que l'analyse du néo-cortex chez les adultes atteints d'X fragile présente une morphologie dendritique spinale anormale suggérant une anomalie de la maturation dendritique postnatale.

### Expérimentation clinique avec un précurseur de la guanosine, à savoir l'inosine

Quarante-six patients ont été traités pendant au moins 3 mois avec 21 résultats favorables dont deux spectaculaires et 25 résultats nuls, sans aucun trouble secondaire signalé :
- Trisomie 21 : sur 19 patients, huit réponses favorables dont deux quasiment spectaculaires chez des malades souffrant de graves troubles du comportement
- Syndrome 4p- : sur 2 patients, 2 résultats favorables
- Syndrome 18q- : sur 4 patients, 2 résultats favorables
- Syndrome de la fragilité de l'X : sur 11 patients, 7 résultats favorables
- Syndrome de Willi Prader : sur 2 patients, 2 résultats favorables
- Syndrome psychotique sans anomalie chromosomique : sur 5 patients, 5 résultats favorables
- Syndrome d'hyperactivité et d'hyperréactivité : sur 2 patients, 2 résultats favorables.

### Indications thérapeutiques

Les effets enregistrés portant sur les troubles du comportement (anxiété, instabilité, agressivité et auto-agressivité, réactions psychotiques ou autistiques etc.), il apparait que la guanosine et ses précurseurs et dérivés immédiats sont susceptibles de rééquilibrer le métabolisme purique perturbé dans de nombreux états de type psychotique. Pour la guanosine les doses prévisibles s'étendent de 5 à 150 mg/kg/jour, et pour les autres précurseurs et dérivés pré-cités, en particulier la xanthosine et l'inosine, les doses d'administration seront comprises entre 20 à 150 mg/kg/jour.

Les médicaments objets de la présente invention peuvent être préparés de manière classique et en soi parfaitement connue. Il peut en particulier s'agir de diverses formes de dosage de la guanosine et/ou de ses dérivés et précurseurs, notamment destinés à l'administration orale, par exemple des comprimés, gélules ou analogues.

Les maladies dont les complications de type psychiatrique sont susceptibles d'être amétiorées par cette médication sont :
- les syndromes chromosomiques entrainant une débilité de l'intelligence (trisomie 21, fragilité de l'X, 4p-, 5p-, 18q- etc.)
- les syndromes de psychose régressive et les syndromes autistiques et, plus généralement, les troubles du comportement survenant au cours de graves maladies du système nerveux central, comme la maladie d'Alzheimer par exemple.

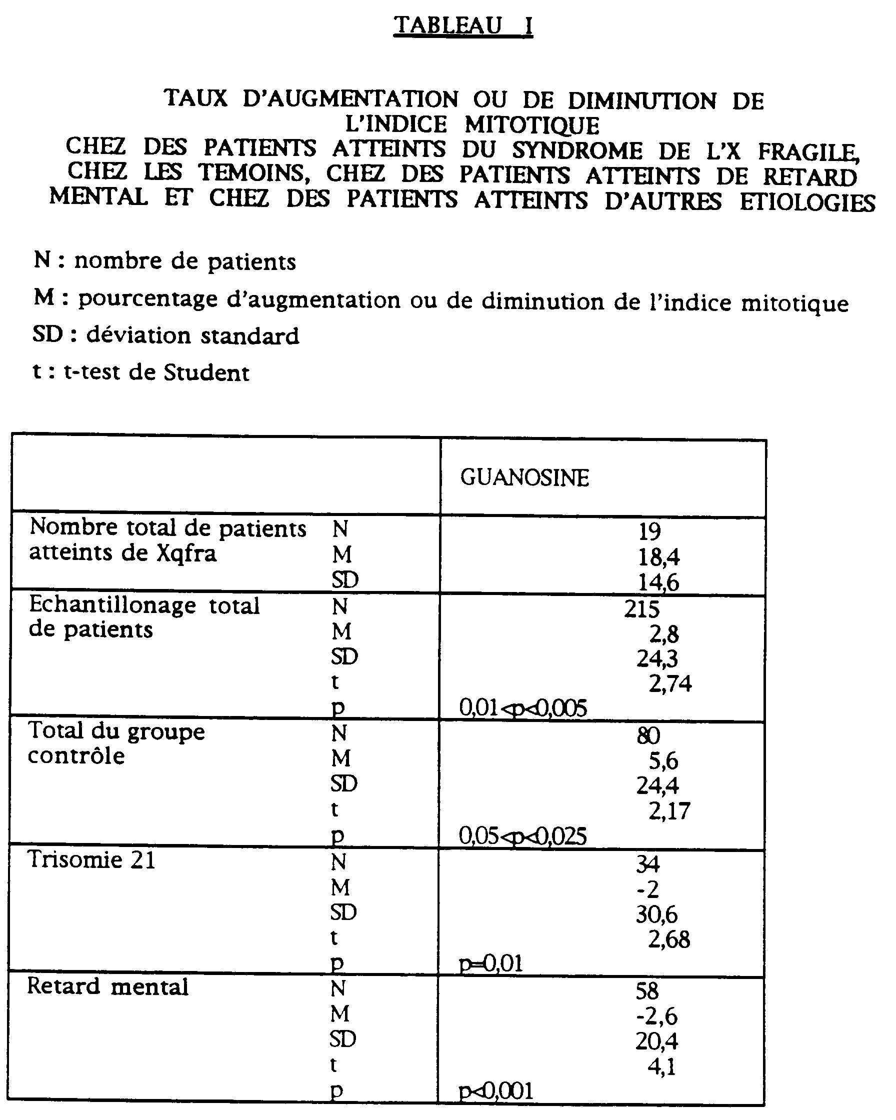

**TABLEAU II**

| **TAUX D'AUGMENTATION OU DE DIMINUTION DE L'INDICE MITOTIQUE CHEZ DES PATIENTS MALES ATTEINTS DU SYNDROME DE L'X FRAGILE, CHEZ DES PATIENTS MALES ATTEINTS DE RETARD MENTAL, CHEZ DES PATIENTS MALES ATTEINTS D'AUTRES ETIOLOGIES ET DANS LE GROUPE DE TEMOINS MALES** | | |
|---|---|---|
| | | GUANOSINE |
| Nombre total de patients mâles atteints de Xqfra | N | 16 |
| | M | 17,6 |
| | SD | 19,7 |
| Echantillonage total de patients mâles | N | 90 |
| | M | -1,4 |
| | SD | 21,8 |
| | t | 3,3 |
| | p | p=0,001 |
| Total du groupe contrôle mâle | N | 29 |
| | M | -1,5 |
| | SD | 19 |
| | t | 3,35 |
| | p | 0,005<p<0,001 |
| Mâles atteints de trisomie 21 | N | 17 |
| | M | -0,5 |
| | SD | 25,5 |
| | t | 2,36 |
| | p | p=0,025 |
| Mâles atteints de retard mental | N | 34 |
| | M | -2,8 |
| | SD | 23 |
| | t | 3,15 |
| | p | 0,005<p<0,001 |

**TABLEAU III**

| **TAUX D'AUGMENTATION OU DE DIMINUTION DE L'INDICE MITOTIQUE CHEZ DES PATIENTS ATTEINTS DU SYNDROME DE L'X FRAGILE ET CHEZ DES PATIENTS ATTEINTS DU SYNDROME DE DOWN** | | |
|---|---|---|
| | | GUANOSINE |
| Nombre total de patients atteints de Xqfra | N | 19 |
| | M | 18,4 |
| | SD | 14,6 |
| Echantillonage de de patients atteints de trisomie 21 et psychotiques | N | 8 |
| | M | 13,4 |
| | SD | 31,3 |
| | t | NS |
| Patients atteints de trisomie 21 sans complications | N | 12 |
| | M | -14 |
| | SD | 21,1 |
| | t | 4,94 |
| | p | p<<0,001 |
| Mâles atteints du syndrome de l'X fragile | N | 16 |
| | M | 17,6 |
| | SD | 15,7 |
| Mâles atteints de trisomie 21 et psychotiques | N | 7 |
| | M | 20,1 |
| | SD | 27,5 |
| | t | 0,3 |
| Mâles atteints de trisomie 21 et psychotiques | N | 7 |
| | M | -19 |
| | SD | 7,3 |
| | t | 5,62 |
| | p | p<<0,001 |

## Revendications

1. Utilisation de la guanosine pour la fabrication de médicaments destinés au traitement des déficits fonctionnels cérébraux.

2. Utilisation selon la revendication 1, caractérisée en ce que lesdits médicaments sont destinés au traitement des syndromes chromosomiques entraînant une débilité de l'intelligence, telle que la trisomie 21 et de la fragilité de l'X.

3. Utilisation selon la revendication 1, caractérisée en ce que lesdits médicaments sont destinés au traitement des troubles du comportement survenant au cours de maladie du système nerveux central, telle que la maladie d'Alzheimer.

4. Utilisation selon la revendication 1, caractérisée en ce que lesdits médicaments sont destinés au traitement de syndromes d'hyperactivité, d'hyperréactivité, d'anxiété, ou de réactions psychotiques ou autistiques.

## Claims

1. Use of guanosine for the manufacture of drugs for the treatment of brain dysfunction.

2. Use according to Claim 1, characterized in that the said drugs are intended for the treatment of chromosomal syndromes causing mental deficiency, such as trisomy 21 and fragile X.

3. Use according to Claim 1, characterized in that the said drugs are intended for the treatment of behavioural disorders occuring in disease of the central nervous system, such as Alzheimer's disease.

4. Use according to Claim 1, characterized in that the said drugs are intended for the treatment of syndromes of hyperactivity, of hyperreactivity, of anxiety, or of psychotic or autistic reactions.

## Patentansprüche

1. Verwendung von Guanosin zur Herstellung von Arzneimitteln zur Behandlung von defizitären cerebralen Funktionen.

2. Verwendung nach Anspruch 1, dadurch gekennzeichnet, daß die Arzneimittel bestimmt sind zur Behandlung von chromosomalen Syndromen, die zu einer Intelligenzschwäche führen, wie Trisomie 21 und Fragilität des X-Chromosoms.

3. Verwendung nach Anspruch 1, dadurch gekennzeichnet, daß die Arzneimittel zur Behandlung von Verhaltensstörungen bestimmt sind, die im Verlauf von Erkrankungen des zentralen Nervensystems auftreten, wie die Alzheimer-Krankheit.

4. Verwendung nach Anspruch 1, dadurch gekennzeichnet, daß die Arzneimittel bestimmt sind zur Behandlung von Hyperaktivitäts-, Hyperreaktivitäts-, Angstsyndromen oder von phsychotischen oder authistischen Reaktionen.
